# EUROPEAN PATENT APPLICATION

(11) **EP 4 700 386 A1**
(43) Date of publication of application: **25.02.2026**
(21) Application number: 25190664.0
(22) Date of filing: 21.07.2025
(51) Int. Cl.: G01N 33/00

(54) **GAS DETECTION APPARATUS AND METHOD FOR AIR FLOW CALIBRATION IN A GAS DETECTION APPARATUS**

(30) Priority: 23.08.2024 CN 202411171246
(71) Applicant: Life Safety Distribution GmbH, 1180 Rolle (CH)
(72) Inventor: LI, Zuohua, Charlotte, 28202 (US); CHEN, Bo, Charlotte, 28202 (US); LIU, Jianli, Charlotte, 28202 (US); MA, Liangliang, Charlotte, 28202 (US)
(74) Representative: Haseltine Lake Kempner LLP

(57) **Abstract**

In accordance with various embodiments of the present disclosure, a gas detection apparatus is provided. In some embodiments, a gas detection apparatus comprises a main body, a selectively attachable and detachable gas sensor cartridge, and a selectively attachable and detachable flow calibration cartridge. The main body comprises a pump to draw air into the main body, through an air chamber, and out of the main body. The gas sensor cartridge comprises a gas sensor and a connection port for providing an airtight connection between the air chamber and the gas sensor. The flow calibration cartridge comprises an air flow sensor and a connection port for providing an airtight connection between the air chamber and the flow calibration cartridge to enable the air to flow between the air chamber and the flow calibration cartridge such that the air flow sensor is exposed to the air to detect an air flow rate.

## Description

### FIELD OF THE INVENTION

Embodiments of the present disclosure generally relate to sensing devices, and, more particularly, to gas detectors.

### BACKGROUND

Many industrial facilities/applications have the potential to produce and/or release one or more gases which may cause a hazardous, sometimes potentially explosive, atmosphere within the facility. Such industrial facilities/applications include, but are not limited to, semiconductor manufacturing facilities, offshore oil and gas platforms, floating production storage and offloading vessels, tankers, onshore oil and gas terminals, refineries, liquified natural gas bottling plants, gas compressor/metering stations, and gas turbine power plants. Such potentially hazardous gases include, but are not limited to, asphyxiant, toxic, flammable, pyrophoric, corrosive, and oxidizing gases. The atmosphere within and around such industrial facilities is typically monitored to detect the presence of such potentially hazardous gases to prevent an accumulation that could result in personal injury and/or property damage.

Conventional gas detectors are often installed in and around such industrial facilities. Such gas detectors often comprise a vacuum pump which draws in ambient air via an input port, directs the air to an air chamber where the air is exposed to a gas sensor element to detect one or more specific gases that may be present in the ambient air. The pump then directs the air from the air chamber out of the gas detector via an output port. For the gas detector to properly work, the flow of air into the detector and air chamber must be within a specified range (or ranges).

To ensure that the flow of air into the detector and air chamber is within the specified range (or ranges), it is important that air flow calibration testing is performed. Such air flow calibration testing is typically performed before the gas detector is shipped from the manufacturer and periodically at the end user's facility. Such air flow calibration testing often requires bulky and expensive test equipment that makes it challenging to perform the testing at the end user's facility.

Through applied effort, ingenuity, and innovation, many of these identified problems have been solved by developing solutions that are included in embodiments of the present disclosure, many examples of which are described in detail herein.

### BRIEF SUMMARY

Various embodiments described herein relate to gas detection apparatuses and methods for air flow calibration testing in gas detection apparatuses.

In accordance with various embodiments of the present disclosure, a gas detection apparatus is provided. In some embodiments, a gas detection apparatus comprises a main body, a gas sensor cartridge selectively attachable to and detachable from the main body, and a flow calibration cartridge selectively attachable to and detachable from the main body. The main body comprises a pump to (i) draw air into the main body, (ii) direct the air through an air chamber in the main body, and (ii) exhaust the air from the main body. The gas sensor cartridge comprises a gas sensor and a connection port for providing an airtight connection between the air chamber of the main body and the gas sensor cartridge when the gas sensor cartridge is selectively attached to the main body to enable the air to flow between the air chamber of the main body and the gas sensor cartridge such that the gas sensor is exposed to the air to detect a presence of one or more predefined gases in the air. The flow calibration cartridge comprises an air flow sensor and a connection port for providing an airtight connection between the air chamber of the main body and the flow calibration cartridge when the flow calibration cartridge is selectively attached to the main body to enable the air to flow between the air chamber of the main body and the flow calibration cartridge such that the air flow sensor is exposed to the air to detect a flow rate of the air.

In some embodiments, the main body further comprises a processing circuitry in communication with the flow calibration cartridge when the flow calibration cartridge is attached to the main body.

In some embodiments, the processing circuitry is configured to receive identification information from the flow calibration cartridge.

In some embodiments, the processing circuitry is configured to receive a detected flow rate of the air from the flow calibration cartridge.

In some embodiments, the processing circuitry is further configured to (i) drive the pump at a pump rate that is expected to draw air into the main body at a first operating flow rate, (ii) receive the detected flow rate of the air from the flow calibration cartridge, and (iii) determine if the received detected flow rate of the air is within a predefined margin of the first operating flow rate.

In some embodiments, if the received detected flow rate of the air is not within the predefined margin of the first operating flow rate, the processing circuitry is further configured provide a test failure message, and, if the received detected flow rate of the air is within the predefined margin of the first operating flow rate, the processing circuitry is further configured to provide a test success message.

In some embodiments, if the received detected flow rate of the air is not within the predefined margin of the first operating flow rate, the processing circuitry is further configured wait a predetermined first timeout period before providing the test failure message.

In some embodiments, if the received detected flow rate of the air is within the predefined margin of the first operating flow rate, the processing circuitry is further configured to drive the pump at a pump rate that is expected to draw air into the main body at a second operating flow rate, (ii) receive the detected flow rate of the air from the flow calibration cartridge, and (iii) determine if the received detected flow rate of the air is within a predefined margin of the second operating flow rate.

In some embodiments, if the received detected flow rate of the air is not within the predefined margin of the second operating flow rate, the processing circuitry is further configured provide a test failure message, and, if the received detected flow rate of the air is within the predefined margin of the second operating flow rate, the processing circuitry is further configured to provide a test success message.

In some embodiments, if the received detected flow rate of the air is not within the predefined margin of the second operating flow rate, the processing circuitry is further configured wait a predetermined second timeout period before providing the test failure message.

In some embodiments, the processing circuitry is further configured to (i) turn off the pump, (ii) receive the detected flow rate of the air from the flow calibration cartridge, and (iii) determine if the received detected flow rate of the air is within a predefined margin of a zero flow rate.

In some embodiments, if the received detected flow rate of the air is not within the predefined margin of the zero flow rate, the processing circuitry is further configured provide a test failure message, and, if the received detected flow rate of the air is within the predefined margin of the zero flow rate, the processing circuitry is further configured to provide a test success message.

In accordance with various embodiments of the present disclosure, a method is provided for calibrating air flow in a gas detection apparatus. In some embodiments, the method comprises when a gas sensor cartridge is selectively removed from a main body of a gas detection apparatus and a flow calibration cartridge is selectively attached to the main body, driving a pump in the main body to draw air (i) into the main body, (ii) through an air chamber in the main body to enable the air to flow between the air chamber of the main body and the flow calibration cartridge such that an air flow sensor in the flow calibration cartridge is exposed to the air to detect a flow rate of the air, and (iii) out of the main body, wherein the pump is driven at a pump rate that is expected to draw air into the main body at a first operating flow rate; receiving a detected flow rate of the air from the flow calibration cartridge; determining if the received detected flow rate of the air is within a predefined margin of the first operating flow rate; determining if the received detected flow rate of the air is within a predefined margin of the first operating flow rate; if the received detected flow rate of the air is not within the predefined margin of the first operating flow rate, providing a test failure message; and if the received detected flow rate of the air is within the predefined margin of the first operating flow rate, providing a test success message. The gas sensor cartridge is selectively attachable to and detachable from the main body. The gas sensor cartridge comprises a gas sensor and a connection port for providing an airtight connection between the air chamber of the main body and the gas sensor cartridge when the gas sensor cartridge is selectively attached to the main body to enable the air to flow between the air chamber of the main body and the gas sensor cartridge such that the gas sensor is exposed to the air to detect a presence of one or more predefined gases in the air. The flow calibration cartridge is selectively attachable to and detachable from the main body. The flow calibration cartridge comprises a connection port for providing an airtight connection between the air chamber of the main body and the flow calibration cartridge when the flow calibration cartridge is selectively attached to the main body.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a block diagram of an example gas detection apparatus in accordance with example embodiments of the present disclosure;
FIG. 2 is a partially transparent perspective view of a gas sensor cartridge of an example gas detection apparatus in accordance with example embodiments of the present disclosure;
FIG. 3 is a partially transparent perspective view of a flow calibration cartridge of an example gas detection apparatus in accordance with example embodiments of the present disclosure;
FIG. 4 is a cutaway side view of an example gas detection apparatus in accordance with example embodiments of the present disclosure; and
FIG. 5 is a flowchart illustrating an example method for calibrating air flow in a gas detection apparatus in accordance with example embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

Various embodiments of the present disclosure overcome the above technical challenges and difficulties and provide various technical improvements and advantages. For example, various embodiments of the present disclosure provide an example gas detection apparatus with a selectively attachable flow calibration cartridge that enables fast and simple calibration of the air flow in the gas detection apparatus without the use of bulky test equipment. Various embodiments of the present disclosure provide an example flow calibration cartridge that is selectively attachable to a gas detection apparatus to enable calibration of the air flow in the gas detection apparatus. Various embodiments of the present disclosure provide an example method for automatically calibrating air flow in a gas detection apparatus using a selectively attachable flow calibration cartridge.

In various embodiments, the selectively attachable flow calibration cartridge has the same general size and shape as a standard gas sensor cartridge that is selectively attachable to and detachable from the example gas detection apparatus for gas detection operations. In this regard, the flow calibration cartridge of various embodiments is selectively attachable to the gas detection apparatus in the same manner and at the same location as the gas sensor cartridge when the gas sensor cartridge is selectively detached from the gas detection apparatus. Such a standard gas sensor cartridge typically comprises a gas sensor and a connection port for connecting to the gas detection apparatus. Such gas sensor cartridges are typically designed to detect the presence of one or more specific potentially dangerous gases (often termed "target gas(es)") in the ambient air.

In various embodiments, the flow calibration cartridge comprises an air flow sensor (which may also be termed a flow meter sensor) and a printed circuit board (PCB) (described below) to enable an air flow calibration process. In various embodiments, the flow calibration cartridge comprises a means to provide identification to a gas detection apparatus, thereby allowing a gas detection apparatus to recognize the flow calibration cartridge once the flow calibration cartridge is attached to the gas detection apparatus. For example, identifying information may be stored in memory on the PCB of the flow calibration cartridge and may be communicated automatically to the gas detection apparatus when the flow calibration cartridge is attached.

In various embodiments, the air flow calibration process involves checking to see if the air drawn into the gas detection apparatus is being drawn in at (or within a suitable margin of) a predetermined air flow rate that is required for the gas detection apparatus to properly detect the target gas(es) (this flow rate may be termed an "operating flow rate"). In various embodiments, a gas detection apparatus may require air be drawn in at two or more different operating flow rates at different times (e.g., for different operating conditions or to detect different gases), in which case the air flow calibration process will check separately that each of the two or more different operating flow rates are within an acceptable margin.

In various embodiments, when the example flow calibration cartridge is selectively attached to the gas detection apparatus, a calibration process (described below) may begin automatically or a user may be prompted to cause the calibration process to start (e.g., by pressing a "calibration start" soft key on the gas detection apparatus.

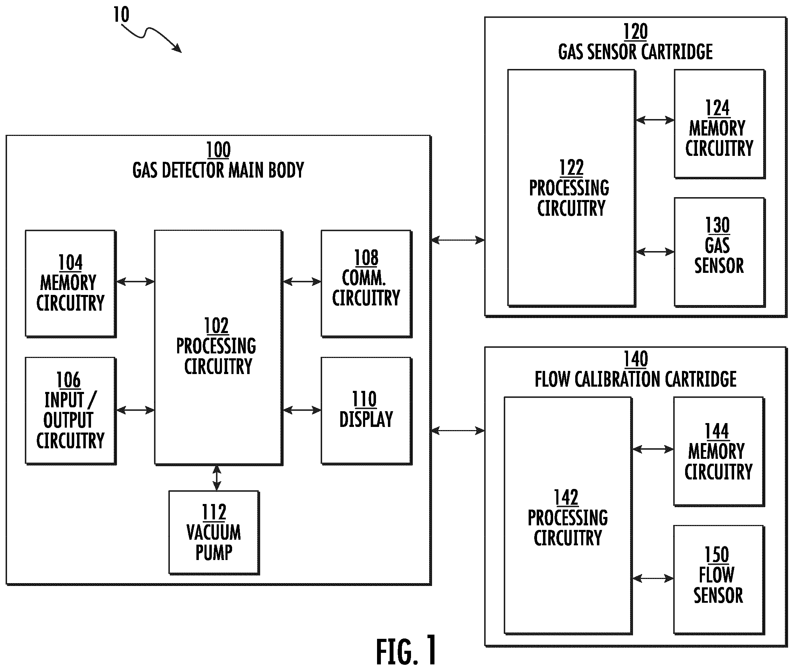

Referring now to FIG. 1, a block diagram of an example gas detection apparatus in accordance with example embodiments of the present disclosure is provided. The example gas detection apparatus 10 of FIG. 1 comprises a gas detector main body 100, a gas sensor cartridge 120, and a flow calibration cartridge 140. While FIG. 1 shows both the gas sensor cartridge 120 and the flow calibration cartridge 140 in communication with the main body 100, only one of the gas sensor cartridge 120 or the flow calibration cartridge 140 will be attached to and in communication with the main body 100 at one time since both the gas sensor cartridge 120 and the flow calibration cartridge 140 attach to the main body 100 at the same attachment point, and use the same electrical connection on the main body 100 for communication.

In the illustrated embodiment of FIG. 1, the main body 100 comprises a processor or processing circuitry 102, memory circuitry 104, input/output circuitry 106, a communications circuitry 108, a display 110, and a vacuum pump 112. In the illustrated embodiment of FIG. 1, the gas sensor cartridge 120 comprises a PCB (not illustrated in FIG. 1) upon which a processor or processing circuitry 122, memory circuitry 124, and a gas sensor 130 are mounted/connected. Any suitable type of gas sensor may be used to detect any one or more target gases. In the illustrated embodiment of FIG. 1, the flow calibration cartridge 140 comprises a PCB (not illustrated in FIG. 1) upon which a processor or processing circuitry 142, memory circuitry 144, and an air flow sensor 150 are mounted/connected. Any suitable type of air flow sensor may be used as long as the air flow sensor is small enough to fit in the housing of the flow calibration cartridge 140. In some embodiments, one or more portions of the example gas detection apparatus 10 are configured to execute and perform the operations described herein.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitry both leverage use of the same processor(s), memory(ies), circuitry(ies), and/or the like to perform their associated functions such that duplicate hardware is not required for each set of circuitry.

Processing circuitry 102, 122, 142 may be embodied in a number of different ways. In various embodiments, the use of the terms "processor" or "processing circuitry" should be understood to include a single core processor, a multi-core processor, multiple processors internal to the example gas detection apparatus 10, and/or one or more remote or "cloud" processor(s) external to the example gas detection apparatus 10. In some example embodiments, processing circuitry 102, 122, 142 may include one or more processing devices configured to perform independently. Alternatively, or additionally, processing circuitry 102, 122, 142 may include one or more processor(s) configured in tandem via a bus to enable independent execution of operations, instructions, pipelining, and/or multithreading.

In an example embodiment, the processing circuitry 102, 122, 142 may be configured to execute instructions stored in the memory circuitry 104, 124, 144 or otherwise accessible to the processor. Alternatively, or additionally, the processing circuitry 102, 122, 142 may be configured to execute hard-coded functionality. As such, whether configured by hardware or software methods, or by a combination thereof, processing circuitry 102, 122, 142 may represent an entity (e.g., physically embodied in circuitry) capable of performing operations according to embodiments of the present disclosure while configured accordingly. Alternatively, or additionally, processing circuitry 102, 122, 142 may be embodied as an executor of software instructions, and the instructions may specifically configure the processing circuitry 102, 122, 142 to perform the various algorithms embodied in one or more operations described herein when such instructions are executed. In some embodiments, the processing circuitry 102, 122, 142 includes hardware, software, firmware, and/or a combination thereof that performs one or more operations described herein.

In some embodiments, the processing circuitry 102, 122, 142 (and/or co-processor or any other processing circuitry assisting or otherwise associated with the processor) is/are in communication with the memory circuitry 104, 124, 144 via a bus for passing information among components of the example gas detection apparatus 10.

Memory or memory circuitry 104, 124, 144 may be non-transitory and may include, for example, one or more volatile and/or non-volatile memories. In some embodiments, the memory circuitry 104, 124, 144 includes or embodies an electronic storage device (e.g., a computer readable storage medium). In some embodiments, the memory circuitry 104, 124, 144 is configured to store information, data, content, applications, instructions, or the like, for enabling the example gas detection apparatus 10 to carry out various operations and/or functions in accordance with example embodiments of the present disclosure.

Input/output circuitry 106 may be included in the example gas detection apparatus 10. In some embodiments, input/output circuitry 106 may provide output to the user and/or receive input from a user. The input/output circuitry 106 may be in communication with the processing circuitry 102, 122, 142 to provide such functionality. The input/output circuitry 106 may comprise one or more user interface(s). In some embodiments, a user interface may include a display (shown separately as display 110) that comprises the interface(s) rendered as a web user interface, an application user interface, a user device, a backend system, or the like. In some embodiments, the input/output circuitry 106 also includes a keyboard, a mouse, a joystick, a touch screen, touch areas, soft keys a microphone, a speaker, or other input/output mechanisms. The processing circuitry 102, 122, 142 and/or input/output circuitry 106 may be configured to control one or more operations and/or functions of one or more user interface elements through computer program instructions (e.g., software and/or firmware) stored on a memory accessible to the processor (e.g., memory circuitry 104, 124, 144, and/or the like). In some embodiments, the input/output circuitry 106 includes or utilizes a user-facing application to provide input/output functionality to a computing device and/or other display associated with a user. In some embodiments, the input/output circuitry 106 one or more indicator lights or the like for providing a user notification (e.g., an alert or warning, as described further below).

Communications circuitry 108 may be included in the example gas detection apparatus 10. The communications circuitry 108 may include any means such as a device or circuitry embodied in either hardware or a combination of hardware and software that is configured to receive and/or transmit data from/to a network and/or any other device, circuitry, or module in communication with the example gas detection apparatus 10. In some embodiments the communications circuitry 108 includes, for example, a network interface for enabling communications with a wired or wireless communications network. Additionally or alternatively, the communications circuitry 108 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 108 may include circuitry for interacting with an antenna(s) and/or other hardware or software to cause transmission of signals via the antenna(s) and/or to handle receipt of signals received via the antenna(s).

The display 110 is any suitable display, typically built into the front of the main body, such as an LCD or LED display.

The vacuum pump 112 is any suitable pump able to draw air into the main body at one or more desired operating flow rates.

In some embodiments, two or more of the sets of circuitry 102-108 are combinable. Alternatively, or additionally, one or more of the sets of circuitry 102-108 perform some or all of the operations and/or functionality described herein as being associated with another circuitry. In some embodiments, two or more of the sets of circuitry 102-108 are combined into a single module embodied in hardware, software, firmware, and/or a combination thereof.

While the description above provides an example gas detection apparatus 10, it is noted that the scope of the present disclosure is not limited to the description above. In some examples, an example gas detection apparatus 10 in accordance with the present disclosure may be in other forms. In some examples, an example gas detection apparatus 10 may comprise one or more additional and/or alternative elements, and/or may be structured differently than that illustrated in FIG. 1.

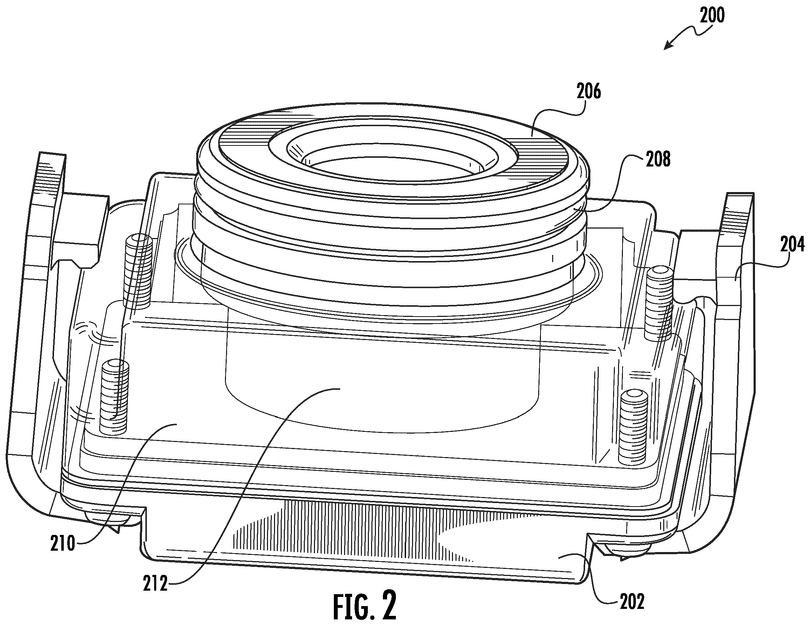

Referring now to FIG. 2, a partially transparent perspective view of a gas sensor cartridge of an example gas detection apparatus is illustrated in accordance with example embodiments of the present disclosure. As seen in FIG. 2, the gas sensor cartridge 200 comprises a housing 202 defining a chamber and having locking arms 204 on opposing sides of the housing 202. The locking arms 204 engage with mating tabs (not illustrated) on the main body of a gas detection apparatus of embodiments of the disclosure when the gas sensor cartridge 200 is selectively attached to the gas detection apparatus. As seen in FIG. 2, the gas sensor cartridge 200 further comprises a PCB 210 within the chamber of the housing 202 upon which a processing circuitry (not illustrated) and a gas sensor 212 are mounted and/or electrically connected. As described above, any suitable gas sensor may be used.

As seen in FIG. 2, the gas sensor cartridge 200 further comprises a connection port 206 with an O-ring 208 to engage with and provide an airtight connection between an air chamber of the main body (described below) and the gas sensor cartridge 200 when the gas sensor cartridge 200 is selectively attached to the main body of a detection apparatus to enable the air to flow between the air chamber of the main body and the gas sensor cartridge 200 such that the gas sensor 212 is exposed to the air to detect a presence of one or more predefined gases in the air.

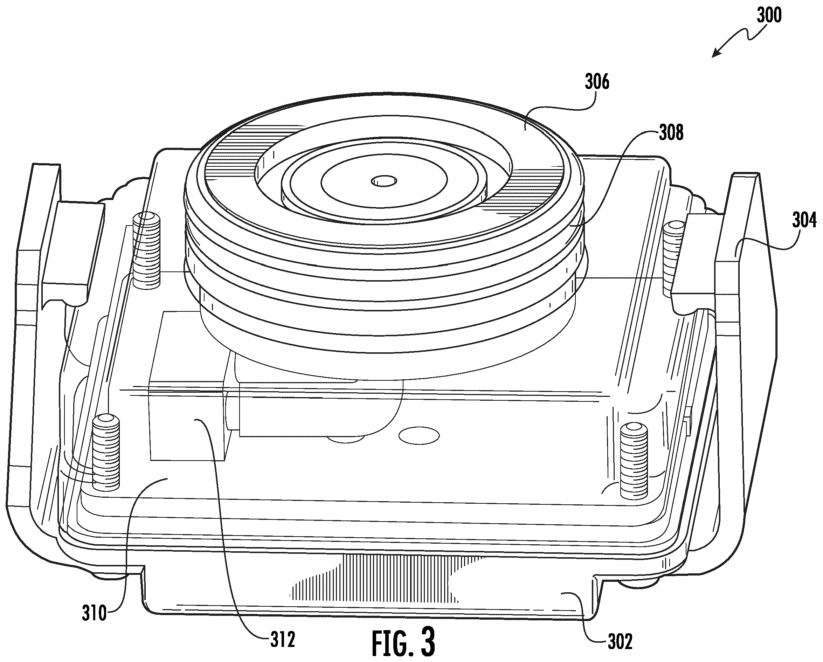

Referring now to FIG. 3, a partially transparent perspective view of a flow calibration cartridge of an example gas detection apparatus is illustrated in accordance with example embodiments of the present disclosure. As seen in FIG. 3, the flow calibration cartridge 300 comprises a housing 302 defining a chamber and having locking arms 304 on opposing sides of the housing 302. The locking arms 304 engage with mating tabs (not illustrated) on the main body of a gas detection apparatus of embodiments of the disclosure when the flow calibration cartridge 300 is selectively attached to the gas detection apparatus. As seen in FIG. 3, flow calibration cartridge 300 further comprises a PCB 310 within the chamber of the housing 302 upon which a processing circuitry (not illustrated) and an air flow sensor 312 are mounted and/or electrically connected. As described above, any suitable air flow sensor may be used.

As seen in FIG. 3, the flow calibration cartridge 300 further comprises a connection port 306 with an O-ring 308 to engage with and provide an airtight connection between an air chamber of the main body (described below) and the flow calibration cartridge 300 when the flow calibration cartridge 300 is selectively attached to the main body of a detection apparatus to enable the air to flow between the air chamber of the main body and the flow calibration cartridge 300 such that the air flow sensor 312 is exposed to the air to determine a flow rate (e.g., in milliliters per minute (ml/min)) of the air.

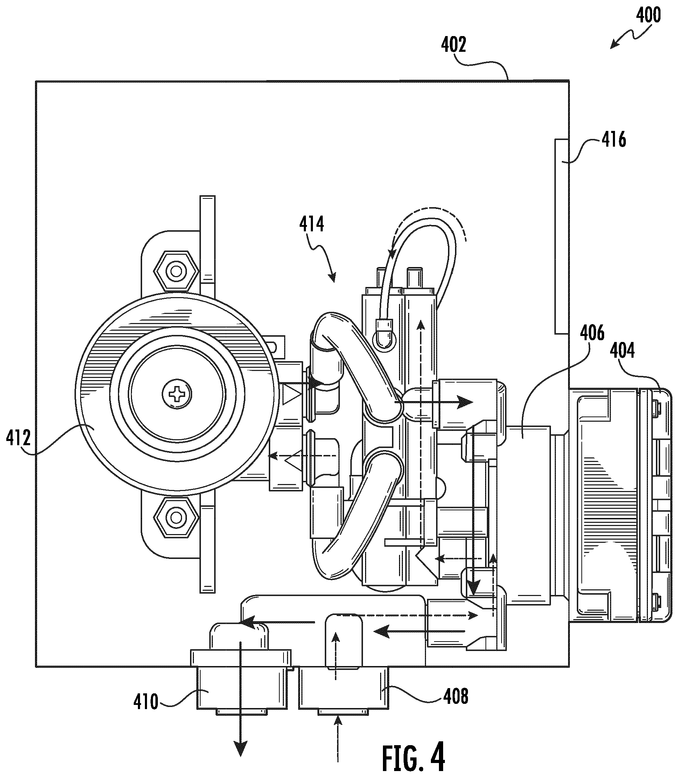

Referring now to FIG. 4, a simplified cutaway side view of an example gas detection apparatus is illustrated in accordance with example embodiments of the present disclosure. As seen in FIG. 4, a gas detection apparatus 400 comprises a main body 402 with a flow calibration cartridge 404 selectively attached thereto. As seen in FIG. 4, the main body 402 comprises an air intake port 408, an air exhaust port 410, an air chamber 406, a vacuum pump 412, an air flow pathway 414 comprising a plurality of tubes, pipes, or the like, and a display 416. In various embodiments during either gas detection operations or during air flow calibration, the vacuum pump 412 is activated to draw ambient air into the main body 402 via the air intake port 408. The drawn in air is circulated, via the vacuum pump 412 and the air flow pathway 414 as indicated by the arrows in FIG. 4, to the air chamber 406 and eventually out of the main body through the air exhaust port 410.

During gas detection operations, when a gas detector cartridge (not illustrated in FIG. 4) is attached to the main body 402, the air in the air chamber 406 flows into the gas detector cartridge and contacts the gas sensor to detect the presence of the target gas(es) in the drawn in air.

During air flow calibration, when the flow calibration cartridge 404 is attached to the main body 402, the air in the air chamber 406 flows into the flow calibration cartridge 404 and contacts the flow sensor (not visible in FIG. 4) to detect the flow rate of the air through the gas detection apparatus 400.

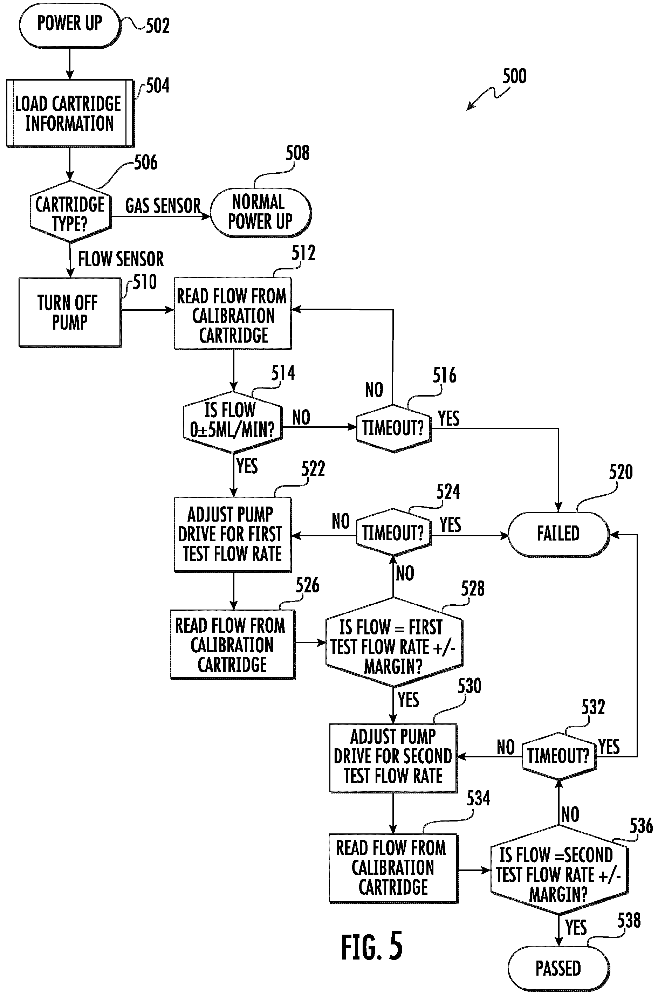

Reference will now be made to FIG. 5 which provides a flowchart illustrating example steps, processes, procedures, and/or operations in accordance with various embodiments of the present disclosure. Various methods described herein, including, for example, methods as shown in FIG. 5, may provide various technical benefits and improvements.

In various embodiments, the example method comprises a method for calibrating air flow in a gas detection apparatus. The example method for calibrating air flow in a gas detection apparatus may be performed automatically when a flow calibration cartridge is attached to a gas detection apparatus or a user may be prompted to cause the calibration process to start (e.g., by pressing a "calibration start" soft key on the gas detection apparatus) when a flow calibration cartridge is attached.

Referring now to FIG. 5, an example method 500 is illustrated. The example method begins at step/operation 502. At step/operation 502, a gas detection apparatus (such as, but not limited to, the example gas detection apparatus 10 described above in connection with FIG. 1) is powered up, such as by a user.

At step/operation 504, a processor (such as, but not limited to, the processing circuitry 102 of the example gas detection apparatus 10 described above in connection with FIG. 1) reads cartridge identifying information from the cartridge that is attached to the gas detection apparatus.

At step/operation 506, the processor determines what type of cartridge is attached to the gas detection apparatus based on the cartridge identifying information read at step/operation 504.

If it is determined at step/operation 506 that the attached cartridge is a gas sensor cartridge (such as, but not limited to, the gas sensor cartridge 120 of the example gas detection apparatus 10 described above in connection with FIG. 1), the method 500 proceeds to step/operation 508 and the gas detection apparatus is powered up for normal gas sensing operations.

If it is determined at step/operation 506 that the attached cartridge is a flow calibration cartridge (such as, but not limited to, the flow calibration cartridge 140 of the example gas detection apparatus 10 described above in connection with FIG. 1), the method 500 proceeds to step/operation 510. At step/operation 510, the processor turns off a vacuum pump (or ensures that the pump is already off) that draws air into the gas detection apparatus (such as, but not limited to, the vacuum pump 112 of the example gas detection apparatus 10 described above in connection with FIG. 1).

At step/operation 512, the processor reads a detected air flow rate from the flow calibration cartridge (as sensed by an air flow sensor (such as, but not limited to, the air flow sensor 150 of the flow calibration cartridge 140 of the example gas detection apparatus 10 described above in connection with FIG. 1)). Because the vacuum pump is off, the detected flow rate should be zero or within an acceptable margin of zero (e.g., +/- 5 ml/min).

At step/operation 514, the processor determines if the detected flow rate is within the acceptable margin of, e.g., +/- 5 ml/min. If it is determined at step/operation 514 that the detected flow rate is not within the acceptable margin of +/- 5 ml/min, the method 500 proceeds to step/operation 516.

At step/operation 516, the processor continues to read the detected air flow and determine if the detected flow rate is within the acceptable margin of +/- 5 ml/min for a predefined timeout period. If the detected flow rate is not within the acceptable margin of +/- 5 ml/min after the predefined timeout period, the method 500 proceeds to step/operation 520 and provides a "test failed" message (such as via a display (such as, but not limited to, the display 110 of the example gas detection apparatus 10 described above in connection with FIG. 1) on the gas detection apparatus).

If it is determined at step/operation 514 that the detected flow rate is within the acceptable margin of +/- 5 ml/min, the method 500 proceeds to step/operation 522. At step/operation 522, the processor drives the vacuum pump at a pump rate that is expected to draw air into the main body at a first operating rate (e.g., 500 ml/min).

At step/operation 526, the processor reads a detected air flow rate from the flow calibration cartridge. At step/operation 528, the processor determines if the detected flow rate is equal to the expected first operating rate or within an acceptable margin (e.g., +/- 5 ml/min).

If it is determined at step/operation 528 that the detected flow rate is not equal to the expected first operating rate or within the acceptable margin, the method 500 proceeds to step/operation 524.

At step/operation 524, the processor continues to read the detected air flow and determine if the detected flow rate is equal to the expected first operating rate or within the acceptable margin for a predefined timeout period. If the detected flow rate is not equal to the expected first operating rate or within the acceptable margin after the predefined timeout period, the method 500 proceeds to step/operation 520 and provides a "test failed" message.

If it is determined at step/operation 528 that the detected flow rate is equal to the expected first operating rate or within the acceptable margin, the method 500 proceeds to step/operation 530.

At step/operation 530, the processor drives the vacuum pump at a pump rate that is expected to draw air into the main body at a second operating rate (e.g., 650 ml/min). At step/operation 534, the processor reads a detected air flow rate from the flow calibration cartridge. At step/operation 536, the processor determines if the detected flow rate is equal to the expected second operating rate or within an acceptable margin (e.g., +/- 5 ml/min).

If it is determined at step/operation 536 that the detected flow rate is not equal to the expected second operating rate or within the acceptable margin, the method 500 proceeds to step/operation 532. At step/operation 532, the processor continues to read the detected air flow and determine if the detected flow rate is equal to the expected second operating rate or within the acceptable margin for a predefined timeout period. If the detected flow rate is not equal to the expected second operating rate or within the acceptable margin after the predefined timeout period, the method 500 proceeds to step/operation 520 and provides a "test failed" message.

If it is determined at step/operation 536 that the detected flow rate is equal to the expected second operating rate or within the acceptable margin, the method 500 proceeds to step/operation 538. At step/operation 538, the processor provides a "test passed" message.

In some embodiments, the method illustrated in FIG. 5 is performed once when a flow calibration cartridge is inserted into a gas detection apparatus and/or on-demand when requested by a user.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A gas detection apparatus comprising:
a main body comprising a pump to (i) draw air into the main body, (ii) direct the air through an air chamber in the main body, and (ii) exhaust the air from the main body;
a gas sensor cartridge selectively attachable to and detachable from the main body, the gas sensor cartridge comprising a gas sensor and a connection port for providing an airtight connection between the air chamber of the main body and the gas sensor cartridge when the gas sensor cartridge is selectively attached to the main body to enable the air to flow between the air chamber of the main body and the gas sensor cartridge such that the gas sensor is exposed to the air to detect a presence of one or more predefined gases in the air; and
a flow calibration cartridge selectively attachable to and detachable from the main body, the flow calibration cartridge comprising an air flow sensor and a connection port for providing an airtight connection between the air chamber of the main body and the flow calibration cartridge when the flow calibration cartridge is selectively attached to the main body to enable the air to flow between the air chamber of the main body and the flow calibration cartridge such that the air flow sensor is exposed to the air to detect a flow rate of the air.

2. The apparatus of claim 1, wherein the main body further comprises a processing circuitry in communication with the flow calibration cartridge when the flow calibration cartridge is attached to the main body; and
wherein the processing circuitry is configured to receive a detected flow rate of the air from the flow calibration cartridge.

3. The apparatus of claim 2, wherein the processing circuitry is further configured to (i) drive the pump at a pump rate that is expected to draw air into the main body at a first operating flow rate, (ii) receive the detected flow rate of the air from the flow calibration cartridge, and (iii) determine if the received detected flow rate of the air is within a predefined margin of the first operating flow rate.

4. The apparatus of claim 3, wherein, if the received detected flow rate of the air is not within the predefined margin of the first operating flow rate, the processing circuitry is further configured provide a test failure message; and
wherein, if the received detected flow rate of the air is within the predefined margin of the first operating flow rate, the processing circuitry is further configured to provide a test success message.

5. The apparatus of claim 4, wherein, if the received detected flow rate of the air is not within the predefined margin of the first operating flow rate, the processing circuitry is further configured wait a predetermined first timeout period before providing the test failure message.

6. The apparatus of claim 3, wherein, if the received detected flow rate of the air is within the predefined margin of the first operating flow rate, the processing circuitry is further configured to drive the pump at a pump rate that is expected to draw air into the main body at a second operating flow rate, (ii) receive the detected flow rate of the air from the flow calibration cartridge, and (iii) determine if the received detected flow rate of the air is within a predefined margin of the second operating flow rate.

7. The apparatus of claim 6, wherein, if the received detected flow rate of the air is not within the predefined margin of the second operating flow rate, the processing circuitry is further configured provide a test failure message; and
wherein, if the received detected flow rate of the air is within the predefined margin of the second operating flow rate, the processing circuitry is further configured to provide a test success message.

8. The apparatus of claim 7, wherein, if the received detected flow rate of the air is not within the predefined margin of the second operating flow rate, the processing circuitry is further configured wait a predetermined second timeout period before providing the test failure message.

9. The apparatus of claim 2, wherein the processing circuitry is further configured to (i) turn off the pump, (ii) receive the detected flow rate of the air from the flow calibration cartridge, and (iii) determine if the received detected flow rate of the air is within a predefined margin of a zero flow rate;
wherein, if the received detected flow rate of the air is not within the predefined margin of the zero flow rate, the processing circuitry is further configured provide a test failure message; and
wherein, if the received detected flow rate of the air is within the predefined margin of the zero flow rate, the processing circuitry is further configured to provide a test success message.

10. A method for calibrating air flow in a gas detection apparatus, the method comprising:
when a gas sensor cartridge is selectively removed from a main body of a gas detection apparatus and a flow calibration cartridge is selectively attached to the main body, driving a pump in the main body to draw air (i) into the main body, (ii) through an air chamber in the main body to enable the air to flow between the air chamber of the main body and the flow calibration cartridge such that an air flow sensor in the flow calibration cartridge is exposed to the air to detect a flow rate of the air, and (iii) out of the main body, wherein the pump is driven at a pump rate that is expected to draw air into the main body at a first operating flow rate;
receiving a detected flow rate of the air from the flow calibration cartridge;
determining if the received detected flow rate of the air is within a predefined margin of the first operating flow rate;
determining if the received detected flow rate of the air is within a predefined margin of the first operating flow rate;
if the received detected flow rate of the air is not within the predefined margin of the first operating flow rate, providing a test failure message; and
if the received detected flow rate of the air is within the predefined margin of the first operating flow rate, providing a test success message;
wherein the gas sensor cartridge is selectively attachable to and detachable from the main body;
wherein the gas sensor cartridge comprises a gas sensor and a connection port for providing an airtight connection between the air chamber of the main body and the gas sensor cartridge when the gas sensor cartridge is selectively attached to the main body to enable the air to flow between the air chamber of the main body and the gas sensor cartridge such that the gas sensor is exposed to the air to detect a presence of one or more predefined gases in the air;
wherein the flow calibration cartridge is selectively attachable to and detachable from the main body; and
wherein the flow calibration cartridge comprises a connection port for providing an airtight connection between the air chamber of the main body and the flow calibration cartridge when the flow calibration cartridge is selectively attached to the main body.

11. The method of claim 10, wherein, if the received detected flow rate of the air is not within the predefined margin of the first operating flow rate, the method further comprises waiting a predetermined first timeout period before providing the test failure message.

12. The method of claim 10, wherein, if the received detected flow rate of the air is within the predefined margin of the first operating flow rate, the method further comprises:
driving the pump at a pump rate that is expected to draw air into the main body at a second operating flow rate;
receiving the detected flow rate of the air from the flow calibration cartridge; and
determining if the received detected flow rate of the air is within a predefined margin of the second operating flow rate.

13. The method of claim 12, wherein, if the received detected flow rate of the air is not within the predefined margin of the second operating flow rate, the method further comprises providing a test failure message; and
wherein, if the received detected flow rate of the air is within the predefined margin of the second operating flow rate, the method further comprises providing a test success message.

14. The method of claim 13, wherein, if the received detected flow rate of the air is not within the predefined margin of the second operating flow rate, the method further comprises waiting a predetermined second timeout period before providing the test failure message.

15. The method of claim 10, wherein the method further comprises:
turning off the pump;
receiving the detected flow rate of the air from the flow calibration cartridge; and
determining if the received detected flow rate of the air is within a predefined margin of a zero flow rate;
wherein, if the received detected flow rate of the air is not within the predefined margin of the zero flow rate, the method further comprises providing a test failure message; and
wherein, if the received detected flow rate of the air is within the predefined margin of the zero flow rate, the method further comprises providing a test success message.
